# EUROPEAN PATENT APPLICATION

(11) **EP 4 732 827 A1**
(43) Date of publication of application: **29.04.2026**
(21) Application number: 25203738.7
(22) Date of filing: 22.09.2025
(51) Int. Cl.: A61K 8/68, A61Q 19/00, A61Q 19/08, A61P 17/06

(54) **MULTIPLE CERAMIDES COMPOSITION AND USE THEREOF**

(30) Priority: 24.10.2024 KR 20240146852; 04.09.2025 KR 20250125788
(71) Applicant: LCS Biotech Co., Ltd., Yongin-si, Gyeonggi-do 17130 (KR)
(72) Inventor: Kim, Jinwook, 16849 Yongin-si, Gyeonggi-do (KR); Lee, Eun Ok, 06517 Seoul (KR)
(74) Representative: Schön, Christoph

(57) **Abstract**

Disclosed herein is a multiple ceramides composition, including one or more of the following (i) to (iii), and one or more of the following (iv) to (v): (i) N-type ceramide, or a stereoisomer thereof, a salt thereof, a solvate thereof, or a hydrate thereof; (ii) A-type ceramide, or a stereoisomer thereof, a salt thereof, a solvate thereof, or a hydrate thereof; (iii) EO-type ceramide, or a stereoisomer thereof, a salt thereof, a solvate thereof, or a hydrate thereof; (iv) O-type ceramide, or a stereoisomer thereof, a salt thereof, a solvate thereof, or a hydrate thereof; and (v) EN-type ceramide, or a stereoisomer thereof, a salt thereof, a solvate thereof, or a hydrate thereof. The multiple ceramides composition exhibits excellent efficacy for the skin.

## Description

### BACKGROUND OF THE INVENTION

### Field of the Invention

The present specification discloses a multiple ceramides composition and a use thereof.

### [Description of Government-Sponsored Research]

A technology disclosed in the present specification is a result of a project of the Korea Health Industry Development Institute (Korean Ministry of Health and Welfare). Specifically, project information is as follows.
Project number: RS-2023-KH141293
Project title: Development of a production process for a high value-added and advanced ceramide complex using a green technology and its commercialization
Name of research project: Project for development of innovation growth for skin health-based technologies (R&D)
Project implementing organization: LCS BIOTECH CO., LTD.
Project period: July 1, 2023 to December 31, 2025

### Description of the Related Art

Human skin is a highly complex and dense tissue that protects the human body from various harmful factors of the external environment. In particular, among various tissues of the skin, a skin barrier present in the outermost layer of the skin serves as a function of a primary human protective shield by preventing evaporation of moisture from the human body and blocking invasion of harmful factors from the external environment, and thus its importance may not be overemphasized. Commonly, the skin barrier is explained by comparing it to a wall made of bricks in a "brick and mortar" model. A portion corresponding to the brick is a keratinocyte which is dead but has biological activity, and a portion corresponding to the mortar is composed of an intercellular lipid layer, which is constituted in a composition ratio of approximately 2:1:1 by weight ratio of ceramide, cholesterol, and free fatty acid. The keratinocyte corresponding to the brick contains a large amount of natural moisturizing factor (NMF) to hold moisture and prevent evaporation, and the intercellular lipid layer has a multi-lamella structure of multiple layers that makes it difficult for moisture or harmful substances to pass through. In a healthy skin barrier, the content of the natural moisturizing factor in the keratinocyte is high, so the keratinocyte does not easily break and maintains an high elastic state, and multiple ceramides in the intercellular lipid layer tightly weave a multilayer lipid layer between barrier cells to form a tightly filled lamellar structure that prevents movement of moisture or substances.

Ceramides, which account for 40 to 50% of the skin barrier lipids, play a key role in skin moisturization and protection. Ceramide is a representative substance of sphingolipid, and performs various structural roles inside cells and simultaneously has important physiological activity necessary for performing normal functions of the cell. All sphingolipids refer to lipids having a sphingoid backbone formed by polymerization of a fatty acid (e.g., palmitic acid) and an amino acid (e.g., serine), and are classified into four types of sphingoids, including dihydrosphingosine, sphingosine, phytosphingosine, and 6-hydroxy sphingosine, depending on a hydroxyl group and an amino group bound to a polar head group of the sphingolipid, and presence or absence of a double bond. The ceramide is formed by binding a fatty acid to an amino group of the four types of sphingoids, and is classified into various classes depending on the type of fatty acid bound.

### SUMMARY OF THE INVENTION

In one aspect, the present invention is directed to providing a composition for improvement of skin barrier function.

In one aspect, the present disclosure is a multiple ceramides composition including one or more of the following (i) to (iii), and one or more of the following (iv) to (v): (i) N-type ceramide, or a stereoisomer thereof, a salt thereof, a solvate thereof, or a hydrate thereof; (ii) A-type ceramide, or a stereoisomer thereof, a salt thereof, a solvate thereof, or a hydrate thereof; (iii) EO-type ceramide, or a stereoisomer thereof, a salt thereof, a solvate thereof, or a hydrate thereof; (iv) O-type ceramide, or a stereoisomer thereof, a salt thereof, a solvate thereof, or a hydrate thereof; and (v) EN-type ceramide, or a stereoisomer thereof, a salt thereof, a solvate thereof, or a hydrate thereof.

In one aspect, the present disclosure is a use for improvement of skin barrier function of the multiple ceramides composition.

In one aspect, the present disclosure is a composition including the multiple ceramides composition as an active ingredient.

In one aspect, by using the present invention, it is possible to perform enhancement of skin barrier function, recovery or improvement of weakened skin barrier function, or treatment or prevention of a disease caused by decrease in skin barrier function.

In one aspect, by using the present invention, it is possible to provide an excellent ceramide composition that may be used in various cosmetics and skin disease formulations.

The disclosed technology may provide a composition capable of mimicking a lamellar structure identical to that of the skin, using the multiple ceramides composition.

The disclosed technology includes various types of ceramides required for construction of the skin barrier, and thus may achieve improvement of skin barrier function.

### BRIEF DESCRIPTION OF THE DRAWINGS

FIG. 1 is a graph showing an effect of N-type ceramide on activity of genes related to skin barrier.
FIG. 2 is a graph showing an effect of A-type ceramide on activity of genes related to skin barrier.
FIG. 3 is a graph showing an effect of EO-type ceramide on activity of genes related to skin barrier.
FIG. 4 is a graph showing an effect of O-type ceramide on activity of genes related to skin barrier.
FIG. 5 is a graph showing an effect of EN-type ceramide on activity of genes related to skin barrier.
FIG. 6 is a graph showing a comparison of effects on activity of genes related to skin barrier of each of: N-type ceramide alone; a ceramide complex including N-type ceramide, A-type ceramide, and EO-type ceramide; and a ceramide complex including N-type ceramide, A-type ceramide, EO-type ceramide, O-type ceramide, and EN-type ceramide.
FIG. 7 and FIG. 8 are results of measuring moisture content after application of ceramide alone and ceramide complex.
FIG. 9 shows the classification of ceramides.

### DETAILED DESCRIPTION OF THE INVENTION

In one aspect, the present invention is a multiple ceramides composition including one or more of the following (i) to (iii); and one or more of the following (iv) to (v): (i) N-type ceramide, or a stereoisomer thereof, a salt thereof, a solvate thereof, or a hydrate thereof; (ii) A-type ceramide, or a stereoisomer thereof, a salt thereof, a solvate thereof, or a hydrate thereof; (iii) EO-type ceramide, or a stereoisomer thereof, a salt thereof, a solvate thereof, or a hydrate thereof; (iv) O-type ceramide, or a stereoisomer thereof, a salt thereof, a solvate thereof, or a hydrate thereof; and (v) EN-type ceramide, or a stereoisomer thereof, a salt thereof, a solvate thereof, or a hydrate thereof.

In one aspect, the multiple ceramides composition may include three or more, four or more, or all five among the above (i) to (v).

The ceramide is one in which a fatty acid is bound to a sphingoid. Specifically, various fatty acids are bound to an amino group of the sphingoid. According to a type of the sphingoid, and according to a type of the fatty acid bound thereto, and according to a binding position of the fatty acid, the ceramide is classified into various classes. The sphingoid includes four types of: sphinganine (G) [dihydrosphingosine], sphingosine, phytosphingosine, and 6-hydroxy sphingosine. The fatty acid may be a non-hydroxyl fatty acid, an α-hydroxyl fatty acid, an esterified ω-hydroxyl fatty acid, or an ω-hydroxyl fatty acid. Meanwhile, there is also a 1-O-acylceramide, in which the fatty acid is further bound to a 1-position OH group of the sphingoid, in addition to the amino group of the sphingoid.

In the present specification, the term "N-type ceramide" refers to a ceramide in which a non-hydroxyl fatty acid (N) is bound to a sphingoid. According to a type of the sphingoid, the N-type ceramide is classified into NG (NDS), NS, NP, and NH ceramides, in which sphinganine (G) [dihydrosphingosine (DS)], sphingosine (S), phytosphingosine (P), and 6-hydroxy sphingosine (H) are respectively bound.

In the present specification, the term "A-type ceramide" refers to a ceramide in which an α-hydroxyl fatty acid (A) is bound to a sphingoid. According to a type of the sphingoid, the A-type ceramide is classified into AG (ADS), AS, AP, and AH ceramides, in which sphinganine (G) [dihydrosphingosine (DS)], sphingosine (S), phytosphingosine (P), and 6-hydroxy sphingosine (H) are respectively bound.

In the present specification, the term "EO-type ceramide" refers to a ceramide in which an esterified ω-hydroxyl fatty acid (EO) is bound to a sphingoid. According to a type of the sphingoid, the EO-type ceramide is classified into EOG (EODS), EOS, EOP, and EOH ceramides, in which sphinganine (G) [dihydrosphingosine (DS)], sphingosine (S), phytosphingosine (P), and 6-hydroxy sphingosine (H) are respectively bound.

In the present specification, the term "O-type ceramide" refers to a ceramide in which an ω-hydroxyl fatty acid (O) is bound to a sphingoid. According to a type of the sphingoid, the O-type ceramide is classified into OG (ODS), OS, OP, and OH ceramides, in which sphinganine (G) [dihydrosphingosine (DS)], sphingosine (S), phytosphingosine (P), and 6-hydroxy sphingosine (H) are respectively bound.

In the present specification, the term "EN-type ceramide" refers to a "1-O-acylceramide", that is, a ceramide in which a fatty acid is further bound to a 1-position OH group of the sphingoid, in addition to an amino group of the sphingoid. According to a type of the sphingoid, the EN-type ceramide is classified into ENG (ENDS), ENS, ENP, and ENH ceramides, in which sphinganine (G) [dihydrosphingosine (DS)], sphingosine (S), phytosphingosine (P), and 6-hydroxy sphingosine (H) are respectively bound.

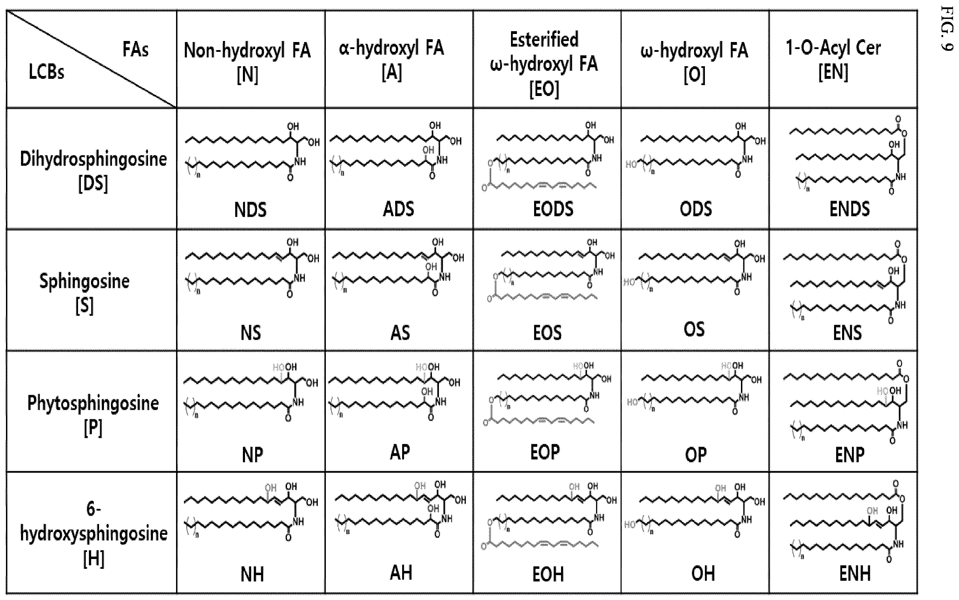

The classification of such ceramides is organized in the Fig. 9. As shown in the above table, when a non-hydroxyl fatty acid is bound to a sphingoid, the ceramide is classified into NG (NDS), NS, NP, and NH, and when an α-hydroxyl fatty acid is bound, the ceramide is named as AG (ADS), AS, AP, and AH. In addition, when an ω-hydroxyl fatty acid is bound, the ceramide is named as OG (ODS), OS, OP, or OH, and when one more fatty acid is connected to a terminal of the fatty acid of the ceramide ODS, OS, OP, or OH, the ceramide is named as EOG (EODS), EOS, EOP, or EOH. Meanwhile, a ceramide having a structure in which one more fatty acid is connected to a 1-position OH group of the ceramide, is named as ENG (ENDS), ENS, ENP, or ENH. By following such naming rules, approximately 20 classes of ceramides exist, and considering the diversity of the length of the fatty acid bound thereto, it becomes possible to obtain hundreds of ceramide combinations or more.

In an embodiment, a weight ratio between one or more of the above (i) to (iii) and one or more of the above (iv) to (v), as a weight ratio among ceramides in the multiple ceramides composition may be from 1.1 to 50 : 1. For example, it may be 1.1:1 or more, 1.5:1 or more, 2:1 or more, 2.5:1 or more, 3:1 or more, 3.5:1 or more, 4:1 or more, 4.5:1 or more, 5:1 or more, 5.5:1 or more, 6:1 or more, 6.5:1 or more, 7:1 or more, 7.5:1 or more, 8:1 or more, 8.5:1 or more, or 9:1 or more. In addition, it may be 50:1 or less, 45:1 or less, 40:1 or less, 35:1 or less, 30:1 or less, 28:1 or less, 26:1 or less, 24:1 or less, 22:1 or less, 20:1 or less, 18:1 or less, 16:1 or less, 14:1 or less, 12:1 or less, 10:1 or less, or 9:1 or less.

In an embodiment, a weight ratio between (i) N-type ceramide, or a stereoisomer thereof, a salt thereof, a solvate thereof, or a hydrate thereof and (ii) A-type ceramide, or a stereoisomer thereof, a salt thereof, a solvate thereof, or a hydrate thereof, as a weight ratio among ceramides in the multiple ceramides composition, may be 1 to 8 : 0 to 1. For example, the A-type may not be included. In addition, when the A-type is included, for example, it may be 1:1 or more, 2:1 or more, 3:1 or more, 4:1 or more, 5:1 or more, or 6:1 or more. In addition, for example, it may be 8:1 or less, 7:1 or less, 6:1 or less, 5:1 or less, 4:1 or less, or 3:1 or less.

In an embodiment, a weight ratio between ((i) N-type ceramide, or a stereoisomer thereof, a salt thereof, a solvate thereof, or a hydrate thereof and (ii) A-type ceramide, or a stereoisomer thereof, a salt thereof, a solvate thereof, or a hydrate thereof) and (iii) EO-type ceramide, or a stereoisomer thereof, a salt thereof, a solvate thereof, or a hydrate thereof (a weight ration of (i)+(ii):(iii)), as a weight ratio among ceramides in the multiple ceramides composition, may be 3:1 to 20:1. For example, it may be 3:1 or more, 3.5:1 or more, 4:1 or more, 4.5:1 or more, 5:1 or more, 5.5:1 or more, 6:1 or more, 6.5:1 or more, 7:1 or more, 7.5:1 or more, 8:1 or more, 8.5:1 or more, 9:1 or more, 9.5:1 or more, or 10:1 or more. In addition, it may be 20:1 or less, 19:1 or less, 18:1 or less, 17:1 or less, 16:1 or less, 15:1 or less, 14:1 or less, 13:1 or less, 12:1 or less, 11:1 or less, or 10:1 or less.

In an embodiment, a weight ratio between (i) N-type ceramide, or a stereoisomer thereof, a salt thereof, a solvate thereof, or a hydrate thereof, (ii) A-type ceramide, or a stereoisomer thereof, a salt thereof, a solvate thereof, or a hydrate thereof, (iii) EO-type ceramide, or a stereoisomer thereof, a salt thereof, a solvate thereof, or a hydrate thereof, (iv) O-type ceramide, or a stereoisomer thereof, a salt thereof, a solvate thereof, or a hydrate thereof, and (v) EN-type ceramide, or a stereoisomer thereof, a salt thereof, a solvate thereof, or a hydrate thereof, as a weight ratio among ceramides in the multiple ceramides composition, may be 30 to 90 : 0 to 26 : 3 to 26 : 1 to 15 : 1 to 15. For example, it may be 30 to 90 : 0 to 26 : 3 to 26 : 1 to 15 : 1 to 15, or 35 to 85 : 0 to 25 : 3 to 24 : 1 to 14 : 1 to 14. Alternatively, it may be 38 to 82 : 0 to 23 : 3 to 22 : 1 to 13 : 2 to 13. Alternatively, it may be 40 to 80 : 0 to 22 : 3 to 21 : 1 to 12 : 3 to 12. Alternatively, it may be 42 to 78 : 0 to 21 : 4 to 20 : 2 to 11 : 4 to 11. Alternatively, it may be 44 to 76 : 0 to 21 : 5 to 19 : 3 to 10 : 4 to 10. Alternatively, it may be 45 to 75 : 0 to 20 : 6 to 19 : 3 to 10 : 4 to 10.

In an embodiment, the multiple ceramides composition may include 30 to 90 wt% of (i) N-type ceramide, or a stereoisomer thereof, a salt thereof, a solvate thereof, or a hydrate thereof, based on a total weight of the multiple ceramides composition. For example, it may be 30 wt% or more, 35 wt% or more, 40 wt% or more, 45 wt% or more, 50 wt% or more, 55 wt% or more, or 60 wt% or more. In addition, it may be 90 wt% or less, 80 wt% or less, 75 wt% or less, 70 wt% or less, 65 wt% or less, or 60 wt% or less.

In an embodiment, the multiple ceramides composition may include 0 to 26 wt% of (ii) A-type ceramide, or a stereoisomer thereof, a salt thereof, a solvate thereof, or a hydrate thereof, based on a total weight of the multiple ceramides composition. For example, it may not be included, or if included, it may be 10 wt% or more, 11 wt% or more, 12 wt% or more, 13 wt% or more, 14 wt% or more, 15 wt% or more, or 16 wt% or more. In addition, it may be 26 wt% or less, 25 wt% or less, 24 wt% or less, 23 wt% or less, 22 wt% or less, 21 wt% or less, or 20 wt% or less.

In an embodiment, the multiple ceramides composition may include 3 to 26 wt% of (iii) EO-type ceramide, or a stereoisomer thereof, a salt thereof, a solvate thereof, or a hydrate thereof, based on a total weight of the multiple ceramides composition. For example, it may be 3 wt% or more, 4 wt% or more, 5 wt% or more, 6 wt% or more, 7 wt% or more, 8 wt% or more, 9 wt% or more, 10 wt% or more, 11 wt% or more, 12 wt% or more, 13 wt% or more, 14 wt% or more, 15 wt% or more, or 16 wt% or more. In addition, it may be 26 wt% or less, 25 wt% or less, 24 wt% or less, 23 wt% or less, 22 wt% or less, 21 wt% or less, 20 wt% or less, 19 wt% or less, 18 wt% or less, 17 wt% or less, 16 wt% or less, 15 wt% or less, or 14 wt% or less.

In an embodiment, the multiple ceramides composition may include 1 to 15 wt% of (iv) O-type ceramide, or a stereoisomer thereof, a salt thereof, a solvate thereof, or a hydrate thereof, based on a total weight of the multiple ceramides composition. For example, it may be 1 wt% or more, 2 wt% or more, 3 wt% or more, 4 wt% or more, 5 wt% or more, 6 wt% or more, 7 wt% or more, 8 wt% or more, 9 wt% or more, or 10 wt% or more. In addition, it may be 15 wt% or less, 14 wt% or less, 13 wt% or less, 12 wt% or less, 11 wt% or less, 10 wt% or less, 9 wt% or less, 8 wt% or less, 7 wt% or less, 6 wt% or less, 5 wt% or less, 4 wt% or less, or 3 wt% or less.

In an embodiment, the multiple ceramides composition may include 1 to 15 wt% of (v) EN-type ceramide, or a stereoisomer thereof, a salt thereof, a solvate thereof, or a hydrate thereof, based on a total weight of the multiple ceramides composition. For example, it may be 1 wt% or more, 2 wt% or more, 3 wt% or more, 4 wt% or more, 5 wt% or more, 6 wt% or more, 7 wt% or more, 8 wt% or more, 9 wt% or more, or 10 wt% or more. In addition, it may be 15 wt% or less, 14 wt% or less, 13 wt% or less, 12 wt% or less, 11 wt% or less, 10 wt% or less, 9 wt% or less, 8 wt% or less, 7 wt% or less, 6 wt% or less, 5 wt% or less, 4 wt% or less, or 3 wt% or less.

In an embodiment, the multiple ceramides composition may include, as N-type ceramides, NP ceramide and NDS ceramide. A weight ratio between the NP ceramide and the NDS ceramide may be 5:1 to 30:1. For example, it may be 5:1 or more, 7:1 or more, 9:1 or more, 10:1 or more, 11:1 or more, 12:1 or more, 13:1 or more, or 14:1 or more. In addition, it may be 30:1 or less, 28:1 or less, 26:1 or less, 24:1 or less, 22:1 or less, 20:1 or less, 18:1 or less, 16:1 or less, or 14:1 or less. Alternatively, it may be approximately 13:1.

In an embodiment, the N-type ceramide may have a fatty acid having a length of C16 to C30.

In an embodiment, the A-type ceramide may have a fatty acid having a length of C16 to C30.

In an embodiment, the EO-type ceramide may be composed of a fatty acid having a length of C24 to C30 primarily bound to a sphingoid. In addition, a fatty acid secondarily bound to a terminal of the primarily bound fatty acid may be linolenic acid and oleic acid.

In an embodiment, the O-type ceramide may be composed of an ω-hydroxyl fatty acid having a length of C24 to C30 bound to a sphingoid.

In an embodiment, the EN-type ceramide may be one in which stearic acid or oleic acid is bound to a 1-position hydroxyl group of the N-type ceramide. The N-type ceramide may be a 1-O-acylceramide represented by Chemical Formula 1 below.

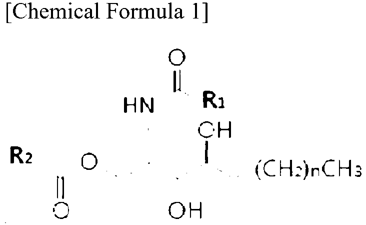

In the above formula, R1 and R2 are each independently a saturated or unsaturated aliphatic chain having C9 to C31, and n is an integer from 10 to 20.

In an embodiment, the ceramide may include a type of sphingoid which is phytosphingosine, sphingosine, sphinganine, or 6-hydroxysphingosine.

Approximately 80% of ceramides of the skin barrier are composed of eight types of ceramides, which are ceramide NG (NDS), NS, NP, NH, ADS, AS, AP, and AH, and these account for the majority of a lamellar layer of the skin barrier. Among these, ceramide NP is the ceramide that is present in the largest amount.

Meanwhile, omega-acylceramides, which are ceramide EOG (EODS), EOS, EOP, and EOH, account for approximately 12% of the skin barrier ceramides, and form a lamella of long periodicity phase having a thick thickness, or are spread across adjacent lamellae like long-chain nails, thereby playing a role of holding the lamella and the lamellar layer.

Ceramides OG (ODS), OS, OP, and OH, which are omega-hydroxy ceramides, also known as protein bound ceramides, form a corneocyte lipid envelope (CLE) that wraps keratinocytes in a single layer during the final differentiation process of the keratinocytes. Skin barrier cells undergo a final differentiation step and die, during which the lipid cell membrane of the keratinocyte disappears and a protein membrane is formed. The keratinocyte comes to have a single lipid layer similar to a lipid cell membrane, due to ceramides bound to involucrin of the corneocyte protein envelope. This not only functions to prevent evaporation of moisture bound to natural moisturizing factors from the keratinocyte, but also plays a role of tightly binding between the keratinocyte and the lamellar layer through the combination between corneocyte lipid envelope (CLE) ceramides and ceramides of the lamellar layer.

Ceramides ENG (ENDS), ENS, ENP, and ENH, which are 1-O-acylceramides having a structure in which one more fatty acid is connected to a 1-position OH group of the ceramide, are positioned in an intermediate layer of the lamellar layer, and extend fatty acid chains to adjacent lamellar layers on both sides, thereby connecting the lamellar layer to another lamellar layer. Such keratinocytes, lamellar layers, and linker type ceramides, which play a role of connecting the lamellar layer and another lamellar layer, are reported to account for approximately 2 to 3% of the total as omega-hydroxy ceramides (protein bound ceramides), and approximately 5% as 1-O-acylceramides (bi-directional linker ceramides).

It has been reported that ceramide is the substance most closely associated with various skin diseases and skin troubles, and changes in ceramides are observed in most skin problems. Recently, due to the advancement of analytical technology, it has become possible to track changes in each individual ceramide.

In one aspect, the present invention may be a cosmetic composition, a food composition, or a pharmaceutical composition including the multiple ceramides composition. In an embodiment, the cosmetic composition, food composition, or pharmaceutical composition may include 0.1 to 10 wt% of the multiple ceramides composition based on a total weight of the cosmetic composition, food composition, or pharmaceutical composition. For example, it may include 0.1 wt% or more, 0.2 wt% or more, 0.3 wt% or more, 0.4 wt% or more, or 0.5 wt% or more. In addition, for example, it may include 10 wt% or less, 9 wt% or less, 8 wt% or less, 7 wt% or less, 6 wt% or less, 5 wt% or less, 4 wt% or less, 3 wt% or less, 2 wt% or less, 1 wt% or less, 0.8 wt% or less, or 0.6 wt% or less.

In an embodiment, the cosmetic composition, food composition, or pharmaceutical composition of the present invention may further include a fatty acid and cholesterol. For example, a weight ratio between a ceramide, a fatty acid, and a cholesterol may be 1:1:1 to 5:1:1.

In an embodiment, the present invention may be a method for improvement of skin barrier function, skin moisturization, improvement of skin itchiness, improvement of skin texture, improvement of skin keratin, improvement of skin density, skin soothing, skin lifting, skin anti-aging, reduction or prevention of skin wrinkles, improvement of skin elasticity, or improvement of skin roughness, and may include administering an effective amount of the ceramide complex to a subject in need thereof.

In a specific example, the cosmetic composition may be a cosmetic composition for improvement of skin barrier function, skin moisturization, improvement of skin itchiness, improvement of skin texture, improvement of skin keratin, improvement of skin density, skin soothing, skin lifting, skin anti-aging, reduction or prevention of skin wrinkles, improvement of skin elasticity, or improvement of skin roughness, including the above-described multiple ceramides composition as an active ingredient.

In an embodiment, the food composition may be a food composition for improvement of skin barrier function, skin moisturization, improvement of skin itchiness, improvement of skin texture, improvement of skin keratin, improvement of skin density, skin soothing, skin lifting, skin anti-aging, reduction or prevention of skin wrinkles, improvement of skin elasticity, or improvement of skin roughness, including the above-described multiple ceramides composition as an active ingredient.

In an embodiment, the pharmaceutical composition may be a pharmaceutical composition for treatment, improvement, or prevention of diseases caused by decrease in skin barrier function, including the above-described multiple ceramides composition as an active ingredient.

In an embodiment, the above disease may be contact dermatitis, allergic dermatitis, or acne. The allergic dermatitis may be an autoimmune skin disease, atopy, or psoriasis. In addition, it may be for recovery or improvement of skin barrier function damaged by ultraviolet rays, fine dust, or the like.

In an embodiment, the present invention relates to a use of the multiple ceramides composition. The use may include both a therapeutic use and a non-therapeutic use. In an embodiment, the use may be for improvement of skin barrier function, skin moisturization, improvement of skin itchiness, improvement of skin texture, improvement of skin keratin, improvement of skin density, skin soothing, skin lifting, skin anti-aging, reduction or prevention of skin wrinkles, improvement of skin elasticity, or improvement of skin roughness.

In the present specification, the term "skin" is a broad concept that includes not only skin but also scalp and hair.

In an embodiment, the multiple ceramides composition may be used as a use for lip moisturization, scalp moisturization, or hair moisturization. In an embodiment, the multiple ceramides composition may be formulated as a formulation for topical application for lip moisturization, scalp moisturization, or hair moisturization.

In an embodiment, the multiple ceramides composition may be orally administered, topically applied, or administered to a subject in need thereof at a daily intake amount, application amount, or administration amount of 0.001 mg to 10 mg, based on a body weight of 60 kg for an adult. For example, the daily administration amount of the multiple ceramides composition, based on a body weight of 60 kg for an adult, may be 0.001 mg or more, 0.005 mg or more, 0.01 mg or more, 0.02 mg or more, 0.03 mg or more, 0.04 mg or more, 0.05 mg or more, 0.06 mg or more, 0.07 mg or more, 0.08 mg or more, 0.09 mg or more, or 0.1 mg or more. Alternatively, based on a body weight of 60 kg for an adult, it may be 10 mg or less, 5 mg or less, 1 mg or less, 0.9 mg or less, 0.8 mg or less, 0.7 mg or less, 0.6 mg or less, 0.5 mg or less, 0.4 mg or less, 0.3 mg or less, 0.2 mg or less, or 0.1 mg or less.

In the present specification, the term "isomer" includes, particularly, optical isomers (e.g., essentially pure enantiomers, essentially pure diastereomers, or mixtures thereof), as well as conformation isomers (i.e., isomers having different angles of one or more chemical bonds), position isomers (in particular, tautomers), or geometric isomers (e.g., cis-trans isomers).

In the present specification, the term "essentially pure", when used in connection with enantiomers or diastereomers, means that a specific compound exemplified by the enantiomer or diastereomer is present in approximately 90% or more, preferably approximately 95% or more, more preferably approximately 97% or more or approximately 98% or more, still more preferably approximately 99% or more, and even more preferably approximately 99.5% or more (w/w).

In the present specification, the term "salt thereof" may be a pharmaceutically acceptable salt. In the present specification, the term "pharmaceutically acceptable" means that it may be approved, has been approved, listed in pharmacopeia, or recognized as general pharmacopeia, as usable in animals, more specifically humans, by avoiding significant toxic effects when used at conventional medicinal dosages, according to governmental or equivalent regulatory authorities.

In the present specification, the term "pharmaceutically acceptable salt" refers to a salt which is pharmaceutically acceptable and possesses the desirable pharmacological activity of the parent compound, according to one aspect of the present invention.

In the present specification, the term "salt thereof" or "pharmaceutically acceptable salt" may include: (1) acid addition salts formed with inorganic acids such as hydrochloric acid, hydrobromic acid, sulfuric acid, nitric acid, phosphoric acid, or organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, , glucoheptonic acid, 3-phenylpropionic acid, trimethylacetic acid, tert-butylacetic acid, lauryl sulfate, gluconic acid, glutamic acid, hydroxynaphthoic acid, salicylic acid, stearic acid, and muconic acid; or (2) salts formed by replacement of an acidic proton present in the parent compound.

In the present specification, the term "hydrate" refers to a compound to which water is bound, and is a broad concept encompassing inclusion compounds in which there is no chemical bonding force between the compound and water.

In the present specification, the term "solvate" refers to a higher-order compound formed between molecules or ions of the solute and molecules or ions of the solvent.

One aspect of the present invention provides a cosmetic composition including the multiple ceramides composition. The cosmetic composition may contain a cosmetologically or dermatologically acceptable medium or base. This may be in any formulation suitable for topical application, for example, may be used in a form of a solution, gel, solid, paste, anhydrous product, an emulsion obtained by dispersing an oil phase in an aqueous phase, an emulsion obtained by dispersing an aqueous phase in an oil phase, a multiple emulsion, a suspension, a microemulsion, a microcapsule, fine granules, an ionic (liposomal) and nonionic vesicle dispersion, a foam, an aerosol further containing a compressed propellant, or a patch. These compositions may be prepared by conventional methods in the relevant field.

The cosmetic composition may be acceptable to contain, in addition to the above-mentioned substance, within a range that does not impair the main effect, preferably, other components that may provide a synergistic effect to the main effect. In addition to the active ingredient of the present invention, other ingredients may be appropriately selected and blended without difficulty by a person skilled in the art according to the formulation type or intended use of other cosmetic compositions. For example, the cosmetic composition of the present invention may include, along with the above active ingredient, other components that are conventionally blended in cosmetic compositions, as needed, and examples thereof may include oil components, moisturizing agents, emollients, surfactants, organic and inorganic pigments, organic powder, ultraviolet absorbers, preservatives, bactericides, antioxidants, stabilizers, thickeners, glycerin, pH adjusters, alcohols, colorants, fragrances, blood circulation promoters, cooling agents, antiperspirants, purified water, and the like. Other blending components that may be included in the cosmetic composition are not limited thereto, and the blending amount of the above components may also be possible within a range that does not impair the purpose and effect of the present invention.

The formulation of the cosmetic composition is not particularly limited, and may be appropriately selected according to the intended purpose. For example, it may be prepared as a formulation of one or more selected from the group consisting of soap-type preparation, softening toner, nutritional toner, essence, nourishing cream, massage cream, pack, gel, makeup base, foundation, powder, lipstick, patch, spray, eye cream, eye essence, cleansing cream, cleansing foam, cleansing water, cleanser, hair shampoo, hair conditioner, hair treatment, hair essence, hair lotion, scalp hair tonic, scalp essence, hair gel, hair spray, hair pack, body lotion, body cream, body oil, and body essence, but is not limited thereto.

One aspect of the present invention provides a pharmaceutical composition including the multiple ceramides composition. Such a pharmaceutical composition may further include, in addition to the active ingredient, pharmaceutical auxiliaries or other therapeutically useful substances, such as preservatives, stabilizers, hydrating agents, emulsifying promoters, salts and/or buffers for adjusting osmotic pressure, diluents (e.g., lactose, dextrose, sucrose, mannitol, sorbitol, cellulose, or glycine), lubricants (e.g., silica, talc, stearic acid and magnesium or calcium salts thereof, or polyethylene glycol), or binders (e.g., magnesium aluminum silicate, starch paste, gelatin, tragacanth, methylcellulose, sodium carboxymethylcellulose, or polyvinylpyrrolidone). In some cases, it may include other pharmaceutical additives such as disintegrants such as starch, agar, alginate or a sodium salt thereof, absorbents, colorants, flavoring agents, or sweeteners.

The pharmaceutical composition may be formulated into various forms of oral or non-oral administration formulations by conventional methods. The non-oral administration formulation may be a formulation, for example, such as a drop formulation, ointment, lotion, gel, cream, spray, suspension, emulsion, suppository, patch, or injection formulation, but is not limited thereto.

According to one aspect of the present invention, the pharmaceutical composition may be administered orally or non-orally, for example, transdermally, intravenously, and the like. The composition conforms to formulations and standards appropriate for each administration method. In particular, in case of intravenous injection, all additives unsuitable therefor are excluded, and the purity of the composition itself is prepared to be very high.

The application amount of the active ingredient may vary depending on the age, gender, body weight, disease, pathological condition, administration route, and judgment of the prescriber of the subject to be treated. The determination of application amount based on these factors falls within the level of a person skilled in the art.

In one aspect of the present invention, the composition may be a food composition. The food composition may be a health functional food composition.

The formulation of the food composition according to one aspect of the present invention is not particularly limited, but may be formulated, for example, as a tablet, granule, powder, liquid preparation such as a drink, caramel, gel, bar, and the like. In addition to the active ingredient, the food composition of each formulation may be prepared by a person skilled in the art without difficulty, depending on the formulation or the purpose of use, with ingredients conventionally used in the art properly selected and combined, and synergistic effects may occur when applied simultaneously with other ingredients.

In the food composition according to one aspect of the present invention, the determination of the administration amount of the active ingredient falls within the level of a person skilled in the art, and its daily administration amount may be, for example, 0.01 mg/kg/day to 10 g/kg/day, but is not limited thereto, and may vary depending on various factors such as the age, health condition, and complications of the subject to be administered.

The food composition according to one aspect of the present invention may be, for example, various types of food products such as chewing gum, caramel products, candies, ice products, snacks, and the like, beverage products such as soft drinks, mineral water, alcoholic beverages, and the like, and health functional foods including vitamins minerals, or the like.

In addition, in one aspect of the present invention, the food composition may include various nutrients, vitamins, minerals (electrolytes), flavoring agents such as synthetic flavoring agents and natural flavoring agents, colorants, and enhancers (cheese, chocolate, etc.), pectic acid and salts thereof, alginic acid and salts thereof, organic acids, protective colloid thickeners, pH adjusters, stabilizers, preservatives, glycerin, alcohol, carbonation agents, and the like used in carbonated beverages. In addition, in one aspect of the present invention, the functional food compositions may include fruit pulp for preparing natural fruit juices, fruit juice beverages, and vegetable beverages. Such components may be used independently or in combination. Although the ratio of such additives is not very critical, in one aspect of the present invention, it is generally included in the range of 0 to approximately 20 parts by weight per 100 parts by weight of the composition.

Hereinafter, the configurations and effects of the present invention will now be described in more detail with reference to examples, comparative examples, and experimental examples. However, the examples, comparative examples, and experimental examples are provided only for illustrative purposes to aid understanding of the present invention, and the scope and extent of the present invention are not to be limited by the examples, comparative examples, and experimental examples described below.

### Example 1) Preparation of Ceramides

Ceramide was respectively prepared through the following preparation methods.

Ceramide NP and ceramide NG were prepared by mixing a fatty acid and phytosphingosine, or a fatty acid and sphinganine, and inducing an N-acylation reaction. As fatty acids, a mixture of palmitic acid, oleic acid, stearic acid, arachidic acid, behenic acid, lignoceric acid, nervonic acid, cerotic acid, and montanic acid was used. Ceramide AP was prepared by inducing an N-acylation reaction between α-hydroxystearic acid and phytosphingosine. Ceramide EOP was prepared by performing N-acylation reaction of omega-hydroxy acid (C20 to C30 hydroxy acid) (omega-hydroxy lignoceric acid, cerotic acid, montanic acid) and phytosphingosine to prepare ceramide OP, and then acylating linolenic acid to the terminal of the ceramide. Ceramide ENP was prepared by acylating the previously prepared ceramide NP with stearic acid. The acylation reaction was performed using the method disclosed in Korean Patent Application No. 10-2016-0004414 (KR10-2017-0084950 A, the entire contents of which are hereby incorporated by this reference), and the ceramide preparation was carried out in accordance with Korean Patent No. 10-2423002, the entire contents of which are hereby incorporated by this reference. The obtained ceramide was separated and purified. The separation and purification was performed by precipitating with addition of 50-fold hexane, followed by filtration, and the precipitate was used.

### Example 2) Preparation 1 of multiple ceramides composition and cream composition including the multiple ceramides composition

A cream composition including the ceramides as active ingredients was prepared.

The cream composition was prepared as a cream in which ceramide NP, ceramide NG (NDS), ceramide AP, ceramide EOP, ceramide OP, and ceramide ENP are incorporated in a base cream including purified water, caprylic/capric triglyceride, stearic acid, oleic acid, cholesterol, hydrogenated lecithin, hexanediol, carbomer, phytosphingosine of 0.015 wt%, and the like, according to the composition shown in Table 1.

**TABLE 1**

| Components | wt% |
|---|---|
| MCT | 4 |
| Glycerine | 3 |
| Lecithin, Emulmetic 950 (HPL) | 3 |
| Phytosqualane | 2 |
| Ceramide or Ceramide complex (multiple ceramides) | 0.5 ~ 0.6% |
| Cholesterol | 0.5 ~ 0.6% |
| Fatty acid (Stearic acid+Oleic acid) | 0.5 ~ 0.6% |
| KMO6 | 2 |
| Carbopol (1%) | 7.5 |
| Tromethamin (17.5%) | 0.3 |
| Phytosphingosine | 0.015 |
| Water | To 100 |

The weight of each of the fatty acid and cholesterol included in the cream was identical to the total weight of the ceramide NP, ceramide AP, ceramide EOP, ceramide OP, and ceramide ENP. As a negative control group, the basic cream, in which all components were identical except that it did not include ceramide, was used. The types and contents of ceramides included in each test group were as follows. Sample 1, which was the negative control group, did not include ceramide, and sample 2 was added with ceramide NP, which is commonly and widely used in the current cosmetic industry, at 0.5%. Sample 3 was a cream to which ceramide NP, ceramide AP, and ceramide EOP were added, and the ceramide content was 0.5% in total, the same as sample 2. In case of sample 4, a cream was prepared by adding ceramide OP and ceramide ENP at 0.025% each to ceramide NP, ceramide AP, and ceramide EOP, and the total ceramide content was prepared as 0.5%, the same as sample 3. The ratio of ceramide NP, ceramide AP, and ceramide EOP in sample 4 was prepared to be identical to that of sample 3. In the sample 5, ceramide OP and ceramide ENP were additionally added at 0.05% each to the sample 3, and the total ceramide content is 0.6%. % refers to wt% in all cases.

**TABLE 2**

| Ceramide | Sample 1 | Sample 2 | Sample 3 | Sample 4 | Sample 5 |
|---|---|---|---|---|---|
| Ceramide NP | - | 0.5% | 0.3% | 0.27% | 0.3% |
| Ceramide AP | - | - | 0.1% | 0.09% | 0.1% |
| Ceramide EOP | - | - | 0.1% | 0.09% | 0.1% |
| Ceramide OP | - | - | - | 0.025% | 0.05% |
| Ceramide ENP | - | - | - | 0.025% | 0.05% |
| Ceramide content in Cream Sample | | 0.5% | 0.5% | 0.5% | 0.6% |

### Example 3) Human Application Test 1

### 1) Test subjects

This human application test was conducted from September to October on female subjects aged between their 20s and 50s, who had no medical history of allergic diseases ,atopic dermatitis, or the like, with the test site limited to the inner side of both the left and right forearms. The amount of cream applied to the skin was approximately 20 mg, which corresponds to a general single-use application amount of cream. Under conditions where the temperature (21.6 ± 2.2°C) and the humidity (48.0 ± 6.5%) of the test location were kept constant, the subjects participated in the test after being stabilized for 30 minutes. In addition, individuals who met any of the following 1 through 4 were excluded from the clinical test.
1. Individuals who have taken any medication that may affect skin response within the past one month (corticosteroids, retinoids, anticancer agents, etc.)
2. Individuals who have severe irritation or allergies to cosmetics, pharmaceuticals, or routine light exposure.
3. Individuals who are simultaneously participating in another clinical test or who have participated in a previous clinical test and sufficient time has not elapsed.
4. Individuals who, due to other diseases or reasons, are deemed inappropriate as subjects by the principal investigator.

### 2) Test method

The basal trans epidermal water loss (Basal TEWL) and skin hydration were measured using Tewameter TM300 (Courage & Khazaka, Germany) and Corneometer CM825 (Courage & Khazaka, Germany), respectively.

### 3) Skin hydration evaluation

On the subject's left forearm, six application sites were respectively selected and marked. After measuring the base line moisture content, the above-prepared samples 1 to 5 were applied twice a day (morning and evening) for four weeks, and the skin moisture content was measured at the second week and fourth week.

### 4) Acute damaged skin barrier recovery rate evaluation

On the subject's right forearm, six application sites were respectively marked to measure TEWL of the base line. Except for the non-application site, acute damage to the skin barrier was induced by continuous tape stripping so that the TEWL became 30 g/h/m² or more, thereby causing damage to the skin barrier. Immediately after the damage, the TEWL was measured, and after applying the above-prepared samples 1 to 5, the TEWL was measured after 4 hours and 8 hours to evaluate the difference in skin barrier recovery level among the respective groups.

### 5) Statistical analysis principle and method

All objective data were statistically analyzed using paired t-tests comparing the vehicle and each of the five samples, and the statistical significance level of the data was set to p-value < 0.01.

The results of the clinical test were as follows.

**TABLE 3**

| Ceramide | Sample 1 | Sample 2 | Sample 3 | Sample 4 | Sample 5 |
|---|---|---|---|---|---|
| Barrier damage recovery rate (4 hours after damage) | 35.1% | 59.2% | 63.6% | 68.3% | 72.5% |
| Barrier damage recovery rate (8 hours after damage) | 46.1% | 70.5% | 75.3% | 82.3% | 85.2% |
| Skin moisture content after 4 weeks | 33.7 | 46.8 | 48.8 | 52.3 | 55.2 |

From the results of the human test of Example 3, it can be confirmed that ceramide recovers acute skin damage to the skin barrier and significantly contributes to skin moisturization. In particular, as seen in samples 2 to 4, compared to ceramide NP alone, the effect of a multiple ceramides composition containing ceramide NP, ceramide AP, and ceramide EOP together is superior. In addition, it can be confirmed that, compared to a multiple ceramides composition containing ceramide NP, ceramide AP, and ceramide EOP, the case of a multiple ceramides composition including all of ceramide NP, ceramide AP, ceramide EOP, ceramide OP, and ceramide ENP is superior in improvement of skin barrier damage and moisturizing effect. In addition, as seen in the comparison between samples 4 and 5, it can be confirmed that the effect increases according to the amount of the ceramide complex contained.

### Example 4) Preparation 2 of multiple ceramides composition and cream composition including the multiple ceramides composition

Sample 6 and sample 7 having different compositions from those in Example 2 were prepared as shown in Table 4 below. Except for the items in Table 4 below, the preparation was carried out in the same manner as in Example 2.

**TABLE 4**

| Ceramide | Sample 1 | Sample 6 | Sample 7 |
|---|---|---|---|
| N-type ceramide | - | 0.375% | 0.375% |
| A-type ceramide | - | 0.041% | - |
| EO-type ceramide | - | 0.083% | 0.065% |
| O-type ceramide | - | - | 0.02% |
| EN-type ceramide | - | - | 0.04% |
| Ceramide content in Cream Sample | | 0.5% | 0.5% |

The N-type ceramide is ceramide NP, the EO-type ceramide is ceramide EOP, the O-type ceramide is ceramide OP, and the EN-type ceramide is ceramide ENP.

### Example 5) Human Application Test 2

The following human application test was performed using the samples prepared in Example 4.

### 1) Test subjects

This human application test was conducted in the same manner as Example 3, except that the test subjects were in their 40s to 50s and the test period was from March to April.

### 2) Test method

It was conducted in the same manner as Example 3.

### 3) Skin hydration evaluation

It was conducted in the same manner as Example 3.

### 4) Acute skin barrier damage recovery test

Except that the barrier damage recovery rate was measured at 3 hours and 6 hours after damage, the test was performed under the same conditions as Example 3.

### 5) Statistical analysis principle and method

It was conducted in the same manner as Example 3.

The results were as follows.

**TABLE 5**

| Ceramide | Sample 1 | Sample 6 | Sample 7 |
|---|---|---|---|
| Barrier damage recovery rate (3 hours after damage) | 27.5% | 31.2% | 38.1% |
| Barrier damage recovery rate (6 hours after damage) | 35.6% | 42.6% | 45.8% |
| Skin moisture content at beginning of test | 27.9 | 28.0 | 27.8 |
| Skin moisture content after 4 weeks of application | 41.1 | 43.5 | 53.1 |
| Increase (%) in skin moisture content after 4 weeks | 147% | 155% | 191% |

In a human clinical test, compared to the case in which N-type ceramide, A-type ceramide, and EO-type ceramide were used, it can be confirmed that skin moisturizaion and skin damage recovery appeared more rapidly in case where EN-type ceramide and O-type ceramide were prescribed together.

### Example 6) Preparation 3 of multiple ceramides composition and cream composition including the multiple ceramides composition

A multiple ceramides composition was prepared based on the method of Example 4 (sample 8). The total content of sphingolipid including ceramide was 0.5%, and a cream containing ceramide NP 0.35%, ceramide EOP 0.046%, ceramide OP 0.02%, ceramide ENP 0.042%, ceramide NDS 0.027%, and phytosphingosine 0.015% was prepared, and various human tests were conducted. Here, the N-type ceramide includes ceramide NP and ceramide NG (NDS) at a weight ratio of approximately 13: 1.

### Example 7) Human Application Test 3

A test was conducted as follows using the test cream composition (sample 8) prepared in Example 6.

### Moisturizing and itchiness improvement effect

A human application test (half-test, no-application comparison, randomized allocation, single-blind) was conducted for evaluation functions to improve itchiness or the like by recovering the function of the skin barrier. The purpose of the test was to evaluate the effect of improving itchiness or the like by recovering the function of the skin barrier. The test was conducted with 34 adult males and females aged 19 to 70 who satisfied the selection and exclusion criteria. In the evaluation items, itchiness evaluation (VAS, Visual Analog Scale) and instrumental evaluation - measurement of transepidermal water loss (Tewameter TM300) - and measurement of skin moisture content (Corneometer CM825), were conducted, and a test subject satisfaction evaluation survey and an expert visual evaluation were conducted in parallel. A total of three measurements were taken: at baseline, 2 weeks, and 4 weeks, during which itchiness evaluation, transepidermal water loss measurement, skin moisture content measurement, photographic evidence, and expert visual evaluation were conducted. The use of the test product was performed twice daily by taking approximately 1 g and gently spreading it over a site where dryness was felt, in correspondence to the Ministry of Food and Drug Safety guidelines (within 3 cm of the antecubital fossa or within 5 cm of the popliteal fossa). In addition, during the human application test period, the use of any cosmetic products (e.g., cosmetics such as moisturizing agents) containing active ingredients that may affect the test results other than the test product was completely prohibited.

The evaluation of itchiness was conducted using a 0 (no itchiness) to 10 (worst itchiness) scale, transepidermal water loss was measured using Tewameter TM300, and skin moisture content was measured using Corneometer CM825. Satisfaction evaluation was rated on a scale of 1 (very excellent) to 5 (very worsened). Expert visual evaluation was conducted by visually evaluating the degree of erythema, scaling, induration, and cracking.

[Result 1] Average itchiness evaluation for 24 hours

**TABLE 6**

| | Cm (Mean ± SD) | |
|---|---|---|
| | Test product | No application |
| Before use | 5.794±0.778 | 5.706±0.813 |
| After 2 weeks of use | 4.356±0.815 | 5.771±0.835 |
| After 4 weeks of use | 1.568±0.759 | 5.897±0.805 |

### [Result 2] Maximum itchiness evaluation for 24 hours

**TABLE 7**

| | Cm (Mean ± SD) | |
|---|---|---|
| | Test product | No application |
| Before use | 6.500±0.681 | 6.450±0.632 |
| After 2 weeks of use | 5.415±0.844 | 6.435±0.606 |
| After 4 weeks of use | 2.138±0.824 | 6.332±0.672 |

### [Result 3] Transepidermal water loss

**TABLE 8**

| | g/m²h (Mean ± SD) | |
|---|---|---|
| | Test product | No application |
| Before use | 16.625±2.381 | 16.518±2.427 |
| After 2 weeks of use | 13.931±1.968 | 16.437±2.364 |
| After 4 weeks of use | 12.776±1.544 | 16.502±2.200 |

### [Result 4] Moisture content

**TABLE 9**

| | A.U. (Mean ± SD) | |
|---|---|---|
| | Test product | No application |
| Before use | 41.069±6.455 | 41.726±6.198 |
| After 2 weeks of use | 58.762±4.683 | 40.839±5.980 |
| After 4 weeks of use | 63.892±4.557 | 41.625±6.111 |

From the above results, it was confirmed that, while the test product was applied for 4 weeks, the skin barrier function was improved and the moisture content of the skin was increased. In addition, as the skin barrier function was improved, the itchiness of the skin was remarkably improved. Therefore, the test product is determined to be a product that, through 4 weeks of use, helps improve itchiness, transepidermal water loss, and skin moisture content, thereby helping to recover the function of the skin barrier and improve itchiness or the like.

### Skin condition improvement effect

By using the above test product, a human application test was conducted on skin moisturization, skin texture, skin elasticity (skin depression mark), skin keratin, skin density, damaged skin soothing and barrier (by external irritation), facial lifting, and overall facial wrinkles in comparison to before use. As a control group, the sample 1 described above was used.

### 1. Measurement of skin moisturization

Skin moisturization was measured on the same right forehead, eye area, nose, cheek, cheekbone, perioral area, and chin site of the test subject before and after use of the test product by using Corneometer CM825 (Courage+Khazaka electronic GmbH, Germany). The measurement parameter was moisture content (arbitrary unit (A.U.)), and an increase in the measured value may indicate there is an improvement effect of skin moisturization.

### 2. Measurement of skin texture

Skin texture was photographed on the same left cheek site of the test subject before and after use of the test product by using Antera 3D CS (Miravex Ltd., Ireland). The photographed image was converted into Textures-small mode and analyzed. The parameter was a Ra (A.U.) value, and a decrease in the value may indicate that there is an improvement effect of skin texture.

### 3. Measurement of skin elasticity (skin depression mark)

Skin elasticity (skin depression mark) was photographed on the same left cheek site of the test subject before pressure, immediately after pressure, and 3 minutes after pressure by using Antera 3D CS (Miravex Ltd., Ireland) before and after use of the test product. The stored images (Depression-Medium mode) were analyzed, and the parameter was volume (mm). This value was used to calculate recoverability, and the result was expressed in percentage (%). An increase in skin depression mark recoverability may indicate improvement, and the formula for calculating skin depression mark recoverability is shown below, with the degree of skin depression mark recovery corrected to an absolute value for clarity.

Skin depression mark recoverability (%) = |(after 3 minutes of pressure - immediately after pressure) / (immediately after pressure - before pressure) X 100|

### 4. Measurement of skin keratin

Skin keratin was collected by tape stripping from the same right cheek site before and after use of the test product using a special film (D-Squame; Cuderm, USA), and analyzed using Visioscan VC20 (Courage+Khazaka electronic GmbH, Germany). The parameter was D.I. (Desquamation Index, %), and a decrease in % value may indicate that there is an improvement effect of skin keratin.

### 5. Measurement of skin density

Skin density was measured on the same right cheek site of the test subject before and after use of the test product using Skin Scanner DUB-USB (TPM taberna pro medicum, Germany). The analysis parameter was Density (%) value, and an increase in the value may indicate that there is an improvement effect of skin density.

### 6. Induction of irritation on damaged skin (due to external irritation)

Induction of irritation on damaged skin (due to external irritation) was carried out by repeatedly attaching and removing (tape stripping) a special film (keratin tape, ADNCS & C BEAUTY, Korea) on the same left forearm area of the test subject to induce skin irritation.

### 7. Measurement of soothing on damaged skin (due to external irritation)

Soothing on damaged skin (due to external irritation) was conducted by photographing the same left forearm area of the test subject before and after use of the test product using Antera 3D CS (Miravex Ltd., Ireland). The photographed image was converted into Hemoglobin mode, and analyzed using Image-pro^{®} plus (Media Cybernetics, USA). The parameter was skin redness (a*). A decrease in the analysis value may indicate that there is a soothing effect on damaged skin (due to external irritation).

### 8. Measurement of barrier of damaged skin (due to external irritation)

Barrier of damaged skin (due to external irritation) was measured by using Vapometer (Delfin Technologies Ltd, Finland) on the same left forearm area of the test subject before and after use of the test product. The parameter was transepidermal water loss (g/m²h), and a decrease in the g/m²h value may indicate that there is an improvement effect of the barrier of damaged skin (due to external irritation).

### 9. Measurement of facial lifting

Facial lifting was measured by photographing the same left face of the test subject at a 30° lateral angle before and after use of the test product using F-ray (BEYOUNG Co., Ltd, Korea), and the photographed image was analyzed using Image-pro^{®} plus (Media Cybernetics, USA). The parameter was angle (°), and a decrease in the analysis value may indicate that there is a facial lifting effect.

### 10. Measurement of overall facial wrinkles

Overall facial wrinkles were measured by photographing the same forehead, glabella, left eye area, right eye area, left nasolabial fold, right nasolabial fold, left perioral area, and right perioral area of the test subject before and after use of the test product using Antera 3D CS (Miravex Ltd., Ireland). The photographed image was converted into Wrinkles-medium mode, and analyzed. The parameters for forehead, glabella, left eye area, and right eye area were Indentation index (A.U.), and the parameters for left nasolabial fold, right nasolabial fold, left perioral area, and right perioral area were depth (mm). A decrease in the values may indicate that there is an improvement effect of overall facial wrinkles.

### [Result 1] Measurement result of skin moisturization (forehead), A.U.

**TABLE 10**

| | A.U. (Mean ± SD) | |
|---|---|---|
| | Test product | control cream |
| Before use | 69.063±6.992 | 69.986±5.873 |
| After 2 weeks of use | 75.267±6.302 | 74.060±5.726 |
| After 4 weeks of use | 79.762±6.799 | 74.495±5.432 |
| Improvement rate (%) after 2 weeks of use | 8.983 | 5.821 |
| Improvement rate (%) after 4 weeks of use | 15.492 | 6.443 |

### [Result 2] Measurement result of skin moisturization (eye area), A.U.

**TABLE 11**

| | A.U. (Mean ± SD) | |
|---|---|---|
| | Test product | control cream |
| Before use | 67.687±6.212 | 69.311±6.603 |
| After 2 weeks of use | 75.562±6.314 | 73.767±6.188 |
| After 4 weeks of use | 80.448±7.178 | 74.184±5.623 |
| Improvement rate (%) after 2 weeks of use | 11.634 | 6.429 |
| Improvement rate (%) after 4 weeks of use | 18.853 | 7.031 |

### [Result 3] Measurement result of skin moisturization (nose), A.U.

**TABLE 12**

| | A.U. (Mean ± SD) | |
|---|---|---|
| | Test product | control cream |
| Before use | 58.028±12.000 | 60.270±6.466 |
| After 2 weeks of use | 65.392±12.884 | 64.844±6.161 |
| After 4 weeks of use | 69.500±12.218 | 65.832±5.705 |
| Improvement rate (%) after 2 weeks of use | 12.690 | 7.589 |
| Improvement rate (%) after 4 weeks of use | 19.770 | 9.228 |

### [Result 4] Measurement result of skin moisturization (cheek), A.U.

**TABLE 13**

| | A.U. (Mean ± SD) | |
|---|---|---|
| | Test product | control cream |
| Before use | 66.573±10.733 | 64.221±7.699 |
| After 2 weeks of use | 74.947±8.620 | 68.419±7.795 |
| After 4 weeks of use | 79.720±7.970 | 69.713±7.724 |
| Improvement rate (%) after 2 weeks of use | 12.579 | 6.537 |
| Improvement rate (%) after 4 weeks of use | 19.748 | 8.552 |

### [Result 5] Measurement result of skin moisturization (cheekbone), A.U.

**TABLE 14**

| | A.U. (Mean ± SD) | |
|---|---|---|
| | Test product | control cream |
| Before use | 69.052±6.968 | 68.949±6.619 |
| After 2 weeks of use | 76.212±5.700 | 73.740±6.390 |
| After 4 weeks of use | 81.683±6.042 | 76.230±6.172 |
| Improvement rate (%) after 2 weeks of use | 10.369 | 6.949 |
| Improvement rate (%) after 4 weeks of use | 18.292 | 10.560 |

### [Result 6] Measurement result of skin moisturization (perioral area), A.U.

**TABLE 15**

| | A.U. (Mean ± SD) | |
|---|---|---|
| | Test product | control cream |
| Before use | 51.405±12.436 | 48.573±10.320 |
| After 2 weeks of use | 58.767±11.504 | 52.690±10.526 |
| After 4 weeks of use | 62.760±11.713 | 53.527±10.338 |
| Improvement rate (%) after 2 weeks of use | 14.322 | 8.476 |
| Improvement rate (%) after 4 weeks of use | 22.089 | 10.199 |

### [Result 7] Measurement result of skin moisturization (chin), A.U.

**TABLE 16**

| | A.U. (Mean ± SD) | |
|---|---|---|
| | Test product | control cream |
| Before use | 66.478±10.313 | 67.306±7.106 |
| After 2 weeks of use | 73.855±9.813 | 71.563±7.471 |
| After 4 weeks of use | 77.667±8.926 | 72.684±7.307 |
| Improvement rate (%) after 2 weeks of use | 11.097 | 6.325 |
| Improvement rate (%) after 4 weeks of use | 16.831 | 7.990 |

### [Result 8] Measurement result of skin texture, Ra (A.U.)

**TABLE 17**

| | Ra(A.U.) (Mean ± SD) | |
|---|---|---|
| | Test product | control cream |
| Before use | 6.784±1.763 | 7.601±2.077 |
| After 2 weeks of use | 5.914±1.396 | 7.478±2.283 |
| After 4 weeks of use | 5.939±1.531 | 7.134±2.193 |
| Improvement rate (%) after 2 weeks of use | 12.824 | - |
| Improvement rate (%) after 4 weeks of use | 12.456 | 6.144 |

### [Result 9] Measurement result of skin elasticity (skin depression mark), %

**TABLE 18**

| | Ra(A.U.) (Mean ± SD) | |
|---|---|---|
| | Test product | control cream |
| Before use | 23.534±20.031 | 28.749±15.564 |
| After 2 weeks of use | 69.517±14.159 | 55.768±34.697 |
| After 4 weeks of use | 87.976±11.987 | 61.084±17.115 |
| Improvement rate (%) after 2 weeks of use | 195.390 | 93.982 |
| Improvement rate (%) after 4 weeks of use | 273.698 | 112.473 |

### [Result 10] Measurement result of skin keratin, %

**TABLE 19**

| | Ra(A.U.) (Mean ± SD) | |
|---|---|---|
| | Test product | control cream |
| Before use | 35.453±3.180 | 37.128±3.095 |
| After 2 weeks of use | 31.148±2.728 | 35.045±2.636 |
| After 4 weeks of use | 28.984±2.242 | 32.914±3.088 |
| Improvement rate (%) after 2 weeks of use | 12.143 | 5.610 |
| Improvement rate (%) after 4 weeks of use | 18.247 | 11.350 |

### [Result 11] Measurement result of skin density, %

**TABLE 20**

| | Ra(A.U.) (Mean ± SD) | |
|---|---|---|
| | Test product | control cream |
| Before use | 7.189±1.793 | 6.755±1.676 |
| After 2 weeks of use | 8.712±1.861 | 7.267±1.823 |
| After 4 weeks of use | 9.886±2.102 | 8.016±1.704 |
| Improvement rate (%) after 2 weeks of use | 21.185 | 7.580 |
| Improvement rate (%) after 4 weeks of use | 37.546 | 18.668 |

### [Result 12] (External irritation) soothing of damaged skin, %

**TABLE 21**

| | Ra(A.U.) (Mean ± SD) | |
|---|---|---|
| | Test product | control cream |
| Before TS | 3.341±3.411 | 3.216±3.306 |
| After TS | 14.933±6.437 | 14.242±7.157 |
| Immediately after use | 4.303±4.415 | 8.622±6.101 |
| Improvement rate (%) | 91.701 | 50.970 |

### [Result 13] (External irritation) Measurement result of damaged skin barrier

**TABLE 22**

| | g/m²/h (Mean ± SD) | |
|---|---|---|
| | Test product | control cream |
| Before TS | 6.022±1.140 | 6.587±1.333 |
| After TS | 11.222±2.099 | 12.295±2.538 |
| Immediately after use | 5.195±1.302 | 7.119±2.185 |
| Improvement rate (%) | 115.904 | 90.680 |

### [Result 14] Measurement result of facial lifting, angle (°)

**TABLE 23**

| | Angle (°) (Mean ± SD) | |
|---|---|---|
| | Test product | control cream |
| Before use | 32.650±3.846 | 31.800±4.467 |
| After 2 weeks of use | 31.598±3.541 | 31.335±4.320 |
| After 4 weeks of use | 31.243±3.717 | 31.120±4.634 |
| Improvement rate (%) after 2 weeks of use | 3.222 | 1.462 |
| Improvement rate (%) after 4 weeks of use | 4.309 | 2.138 |

### [Result 15] Measurement result of overall facial wrinkle (forehead), Indentation Index (A.U.)

**TABLE 24**

| | Indentation Index(A.U.) (Mean ± SD) | |
|---|---|---|
| | Test product | control cream |
| Before use | 12.818±3.321 | 12.245±3.301 |
| After 2 weeks of use | 11.442±3.179 | 11.462±2.852 |
| After 4 weeks of use | 10.883±3.035 | 11.409±3.187 |
| Improvement rate (%) after 2 weeks of use | 10.735 | 6.394 |
| Improvement rate (%) after 4 weeks of use | 15.096 | 6.827 |

### [Result 16] Measurement result of overall facial wrinkle (glabella), Indentation Index (A.U.)

**TABLE 25**

| | Indentation Index(A.U.) (Mean ± SD) | |
|---|---|---|
| | Test product | control cream |
| Before use | 15.252±3.324 | 16.593±4.077 |
| After 2 weeks of use | 13.423±3.419 | 15.687±4.190 |
| After 4 weeks of use | 12.804±3.320 | 14.836±3.997 |
| Improvement rate (%) after 2 weeks of use | 11.992 | 5.460 |
| Improvement rate (%) after 4 weeks of use | 16.050 | 10.589 |

### [Result 17] Measurement result of overall facial wrinkle (left eye area), Indentation Index (A.U.)

**TABLE 26**

| | Indentation Index(A.U.) (Mean ± SD) | |
|---|---|---|
| | Test product | control cream |
| Before use | 42.889±6.146 | 43.063±6.852 |
| After 2 weeks of use | 39.551±5.822 | 41.321±5.767 |
| After 4 weeks of use | 38.482±7.523 | 40.937±6.551 |
| Improvement rate (%) after 2 weeks of use | 7.783 | 4.045 |
| Improvement rate (%) after 4 weeks of use | 10.275 | 4.937 |

### [Result 18] Measurement result of overall facial wrinkle (right eye area), Indentation Index (A.U.)

**TABLE 27**

| | Indentation Index(A.U.) (Mean ± SD) | |
|---|---|---|
| | Test product | control cream |
| Before use | 42.838±6.300 | 42.058±4.003 |
| After 2 weeks of use | 38.883±7.658 | 40.545±3.504 |
| After 4 weeks of use | 37.166±7.071 | 39.471±3.868 |
| Improvement rate (%) after 2 weeks of use | 9.232 | 3.597 |
| Improvement rate (%) after 4 weeks of use | 13.241 | 6.151 |

### [Result 19] Measurement result of overall facial wrinkle (left nasolabial fold), depth (mm)

**TABLE 28**

| | depth(mm) (Mean ± SD) | |
|---|---|---|
| | Test product | control cream |
| Before use | 0.123±0.041 | 0.199±0.032 |
| After 2 weeks of use | 0.094±0.029 | 0.102±0.032 |
| After 4 weeks of use | 0.084±0.023 | 0.100±0.031 |
| Improvement rate (%) after 2 weeks of use | 23.577 | 14.286 |
| Improvement rate (%) after 4 weeks of use | 31.707 | 15.966 |

### [Result 20] Measurement result of overall facial wrinkle (right nasolabial fold), depth (mm)

**TABLE 29**

| | depth(mm) (Mean ± SD) | |
|---|---|---|
| | Test product | control cream |
| Before use | 0.116±0.042 | 0.120±0.041 |
| After 2 weeks of use | 0.087±0.036 | 0.106±0.038 |
| After 4 weeks of use | 0.082±0.033 | 0.101±0.037 |
| Improvement rate (%) after 2 weeks of use | 25.000 | 11.667 |
| Improvement rate (%) after 4 weeks of use | 29.310 | 15.833 |

### [Result 21] Measurement result of overall facial wrinkle (left perioral area), depth (mm)

**TABLE 30**

| | depth(mm) (Mean ± SD) | |
|---|---|---|
| | Test product | control cream |
| Before use | 0.265±0.092 | 0.282±0.109 |
| After 2 weeks of use | 0.238±0.092 | 0.269±0.106 |
| After 4 weeks of use | 0.218±0.083 | 0.253±0.109 |
| Improvement rate (%) after 2 weeks of use | 10.189 | 4.610 |
| Improvement rate (%) after 4 weeks of use | 17.736 | 10.284 |

### [Result 22] Measurement result of overall facial wrinkle (right perioral area), depth (mm)

**TABLE 31**

| | depth(mm) (Mean ± SD) | |
|---|---|---|
| | Test product | control cream |
| Before use | 0.264±0.111 | 0.280±0.089 |
| After 2 weeks of use | 0.234±0.102 | 0.266±0.086 |
| After 4 weeks of use | 0.219±0.099 | 0.259±0.094 |
| Improvement rate (%) after 2 weeks of use | 11.364 | 5.000 |
| Improvement rate (%) after 4 weeks of use | 17.045 | 7.500 |

As a result of this clinical test, by the application of the test product, skin moisturization was statistically significantly improved after 2 weeks of use and after 4 weeks of use in all sites of the face, namely, the forehead, eye area, nose, cheek, cheekbone, perioral area, and chin. In the comparison between the test product and the control cream, significant improvement was also confirmed. Skin texture improvement effect was also statistically significantly improved after 2 weeks of use and after 4 weeks of use compared to before use. Skin elasticity (skin depression mark) was also statistically significantly improved after 2 weeks of use and after 4 weeks of use compared to before use, and skin keratin was also statistically significantly improved after 2 weeks of use and after 4 weeks of use compared to before use. In terms of skin density as well, statistically significant improvement was observed after 2 weeks of use and after 4 weeks of use compared to before use, and the soothing effect of damaged skin was also statistically significantly increased after TS compared to before TS, and statistically significant improvement was confirmed immediately after use compared to after TS. In addition, it was confirmed that facial lifting, overall facial wrinkles, wrinkles around the eyes, nasolabial wrinkles, and wrinkles in perioral area were all statistically significantly improved after 2 weeks of use and after 4 weeks of use compared to before use.

### Example 8) Preparation 4 of ceramide composition and cream composition including the ceramide composition

Ceramide compositions were prepared in the same manner as in Example 2, except for using ceramide NP alone, ceramide NG (NDS) alone, and a ceramide complex of NP and NG (NDS) as the ceramide.

**TABLE 32**

| Ceramide | Sample 1 | Sample 9 | Sample 10 | Sample 11 | Sample 12 | Sample 13 |
|---|---|---|---|---|---|---|
| NP | - | 0.5% | | 0.25% | 0.33% | 0.4% |
| NG | - | | 0.5% | 0.25% | 0.17% | 0.1% |

### Example 9) Human Application Test 4

Skin hydration evaluation was performed on 1) test subjects described in Example 3 using samples 9 to 13 of Example 8.

Skin hydration evaluation

Six application sites were respectively marked and selected on the left forearm of the subject. After measuring the baseline moisture content, the above samples were applied twice daily (morning and evening) for two weeks, and the skin moisture content was measured at the first week and second week.

Skin hydration was measured using Corneometer CM825 (Courage & Khazaka, Germany).

After two weeks, the application of samples was discontinued, and for three days the moisture content of the treated area was measured to measure the degree of moisturization retained.

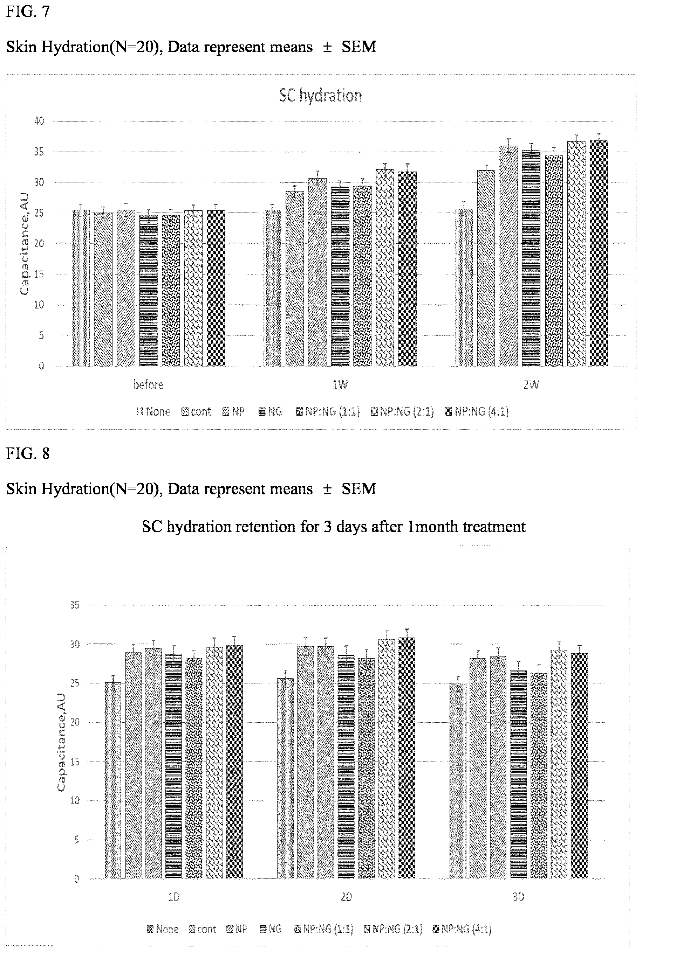

The results are shown in FIG. 7 and FIG. 8.

### Example 10) Analysis of Effect of Each Ceramide on activity of Genes Related to Skin Barrier

The effect of ceramides NP, AP, EOP, OP, and ENP on the activity of genes related skin barrier was analyzed. In addition, as ceramide complexes, the effects of the above-prepared sample 3 and sample 4 were also analyzed.

### Cell culture and RNA extraction

Human epidermal keratinocytes (neonatal, (HEKn), Thermo Fisher Scientific, USA) were added at 1×10⁵ cells per well into a 12-well plate and cultured for 24 hours at 37°C under 5% CO₂ aerated conditions. After treating with the ceramides prepared in the above examples at a 5 µM concentration, the cells were further cultured for 24 hours at 37°C under 5% CO₂ aerated conditions. After removing the media and washing once with PBS buffer, RNA was extracted using the NucleoSpin RNA Plus kit (Germany). The conditions were as follows: 350 µL of LBP (lysate buffer) was added to each well and scraped with a scraper, and the cell lysate was collected into the NucleoSpin gDNA Removal Column (yellow ring), followed by centrifugation at 11,000g for 30 seconds. 100 µL of BS (Binding Solution) was added to the collected solution and mixed, and the mixture was transferred to a NucleoSpin RNA Plus Column (blue ring) and centrifuged at 11,000g for 15 seconds. 200 µL of Buffer WB1 (washing buffer) was added to the column, and washed by centrifugation at 11,000g for 15 seconds, and 600 µL of Buffer WB2 (washing buffer) was further added and washed by centrifugation at 11,000g for 15 seconds. Finally, 250 µL of Buffer WB2 (washing buffer) was added, centrifuged at 11,000g for 2 minutes to wash. Each step above is a process of removing residual proteins, lipids, and DNA. 20 µL of RNase-free H₂O was added, and centrifugation was performed at 11,000g for 1 minute. (Twice) the extracted RNA was collected into sterilized microtubes, and RNA was quantified. RNA absorbance was measured at 260 nm and 280 nm, and the amount of RNA was calculated. In this case, RNase-free water was used as the blank.

### Synthesis of cDNA from RNA

To RNA correction value + DEPC water correction value (total 11 µL), 1 µL of oligo-dT primer was added and collected by centrifugation, then heated at 65°C for 5 minutes. After cooling with centrifugation, for each sample, 4 µL of buffer + 2 µL of dNTP mix + 1 µL of RNase inhibitor + 1 µL of RNA transcriptase (total 20 µL) was added, and allowed to stand at room temperature for 5 minutes, followed by enzyme reaction at 42°C for 1 hour. The enzyme stop reaction was performed at 70°C for 5 minutes.

### RT-PCR

The RT-PCR mixture was as follows: 2 µL of synthesized cDNA + 0.5 µL of primer mix (10 µM) + 12.5 µL of TB Green^{®} Premix Ex Taq, and the remainder was filled with distilled water to prepare a total volume of 25 µL. The operating conditions were: heating once at 95°C for 10 seconds, followed by cycling 95°C for 5 seconds, and then 60°C for 1 minute for 40 cycles. The primer sequences used in this case were indicated below.

**TABLE 33**

| Function | Relative gene | Primer sequences | Sequence number |
|---|---|---|---|
| Ceramide Synthesis | CER3 (F) | ACA TTC CAC AAG GCA ACC ATT G | 1 |
| | CER3 (R) | CTC TTG ATT CCG CCG ACT CC | 2 |
| | SPT-1 (F) | GCGCGCTACTTGGAGAAAGA | 3 |
| | SPT-1 (R) | TGTTCCACCGTGACCACAAC | 4 |
| | ELOVL4 (F) | TAG TGT CCA CGG CAC TCA AC | 5 |
| | ELOVL4 (R) | AAG TAC CAC CAC AGA GCA GC | 6 |
| Differentiation | FLG (F) | GGC ACT GAA AGG CAA AAA GG | 7 |
| | FLG (R) | AAA CCC GGA TTC ACC ATA ATC A | 8 |
| | IVL (F) | CCA TCA GGA GCA AAT GAA ACA G | 9 |
| | IVL (R) | GCT CGA CAG GCA CCT TCT G | 10 |
| | CASP14 (F) | GAC CTG GAT GCT CTG GAA CAC A | 11 |
| | CASP14 (R) | GAA TCG ATG GCC TGC TGG A | 12 |

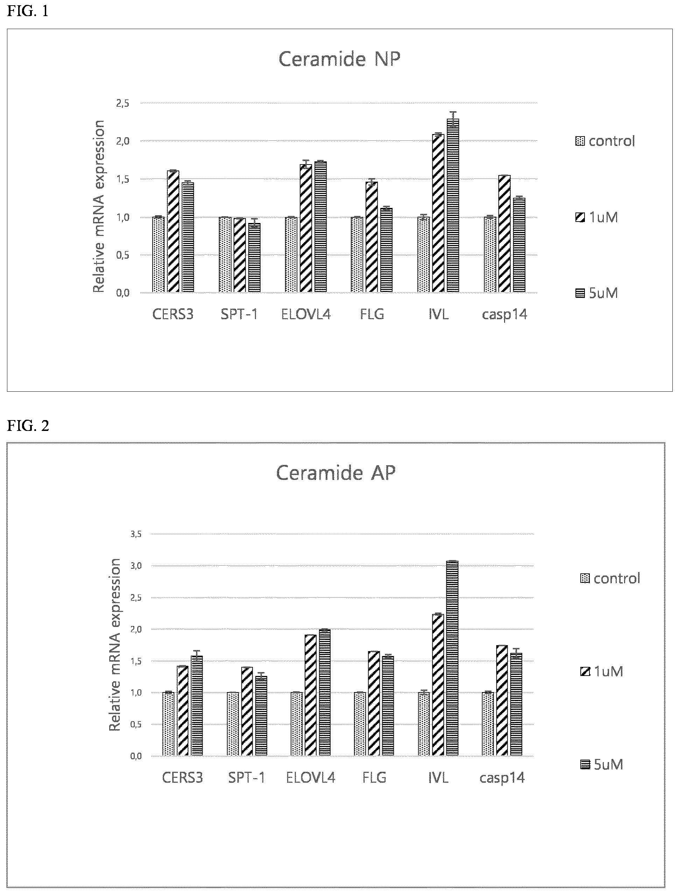

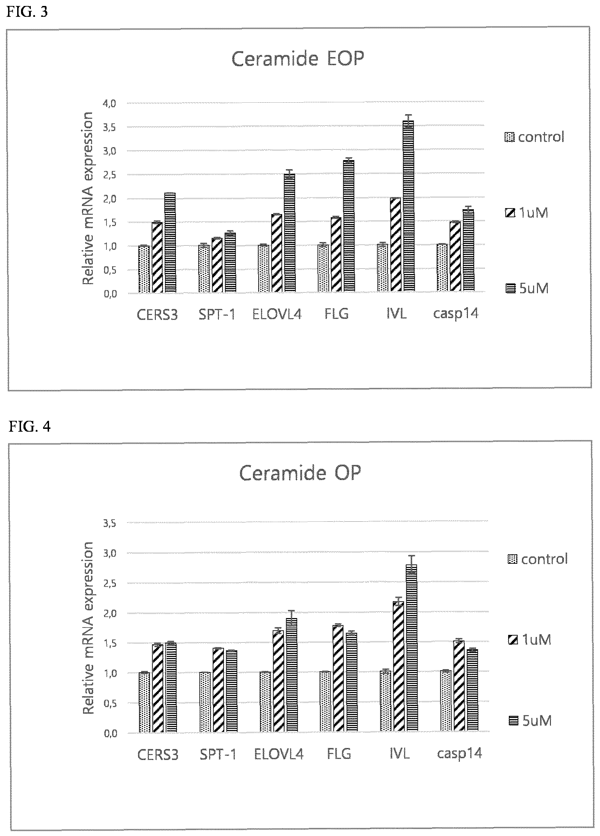

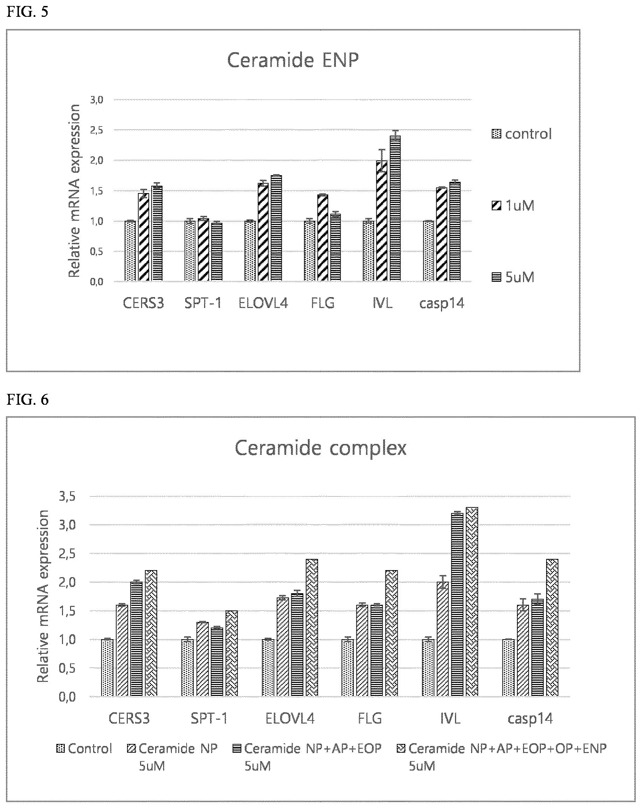

The result was shown in FIG. 1 to FIG. 6. As shown in FIG. 1 to FIG. 6, as a result of analyzing the effect on ceramide metabolism and skin differentiation using skin cells for each class of phytosphingosine-based ceramides, although there was a difference in degree between ceramides, it was confirmed that most had a positive effect on skin barrier formation. In addition, compared to ceramide NP alone, it was also confirmed that, in case of ceramide complex, the effect significantly increased.

## Claims

1. A multiple ceramides composition, comprising one or more of the following (i) to (iii), and one or more of the following (iv) to (v):
(i) N-type ceramide, or a stereoisomer thereof, a salt thereof, a solvate thereof, or a hydrate thereof;
(ii) A-type ceramide, or a stereoisomer thereof, a salt thereof, a solvate thereof, or a hydrate thereof;
(iii) EO-type ceramide, or a stereoisomer thereof, a salt thereof, a solvate thereof, or a hydrate thereof;
(iv) O-type ceramide, or a stereoisomer thereof, a salt thereof, a solvate thereof, or a hydrate thereof; and
(v) EN-type ceramide, or a stereoisomer thereof, a salt thereof, a solvate thereof, or a hydrate thereof.

2. The multiple ceramides composition of claim 1, wherein the multiple ceramides composition comprises three or more of the (i) to (v).

3. The multiple ceramides composition of any one of claims 1-2, wherein the multiple ceramides composition comprises four or more of the (i) to (v).

4. The multiple ceramides composition of any one of claims 1-3, wherein the multiple ceramides composition comprises the (i), (iii), (iv), and (v).

5. The multiple ceramides composition of any one of claims 1-4, wherein the multiple ceramides composition comprises all of the (i) to (v).

6. The multiple ceramides composition of any one of claims 1-5, wherein a weight ratio between one or more of the (i) to (iii) and one or more of the (iv) to (v) is 1.1 to 50 : 1.

7. The multiple ceramides composition of any one of claims 1-6, wherein a weight ratio between (i) N-type ceramide, or a stereoisomer thereof, a salt thereof, a solvate thereof, or a hydrate thereof and (ii) A-type ceramide, or a stereoisomer thereof, a salt thereof, a solvate thereof, or a hydrate thereof, as a weight ratio among ceramides in the multiple ceramides composition, is 1-8 : 0-1.

8. The multiple ceramides composition of any one of claims 1-7, wherein a weight ratio between (i) N-type ceramide, or a stereoisomer thereof, a salt thereof, a solvate thereof, or a hydrate thereof, (ii) A-type ceramide, or a stereoisomer thereof, a salt thereof, a solvate thereof, or a hydrate thereof, (iii) EO-type ceramide, or a stereoisomer thereof, a salt thereof, a solvate thereof, or a hydrate thereof, (iv) O-type ceramide, or a stereoisomer thereof, a salt thereof, a solvate thereof, or a hydrate thereof, and (v) EN-type ceramide, or a stereoisomer thereof, a salt thereof, a solvate thereof, or a hydrate thereof, as a weight ratio among ceramides in the multiple ceramides composition, is 30 to 90 : 0 to 26 : 3 to 26 : 1 to 15 : 1 to 15.

9. The multiple ceramides composition of any one of claims 1-8, wherein the ceramides are ceramides comprising phytosphingosine, sphingosine, sphinganine, or 6-hydroxysphingosine, as sphingoids.

10. The multiple ceramides composition of any one of claims 1-9, wherein the N-type ceramide comprises ceramide NP and ceramide NDS.

11. A cosmetic composition, comprising the multiple ceramides composition according to any one of claims 1-10.

12. The cosmetic composition of claim 11, wherein the cosmetic composition comprises the multiple ceramides composition in an amount of 0.1 to 10 wt% based on a total weight of the cosmetic composition.

13. A use of the multiple ceramides composition of any one of claims 1-10 for improvement of skin barrier function, skin moisturization, improvement of skin itchiness, improvement of skin texture, improvement of skin keratin, improvement of skin density, skin soothing, skin lifting, skin anti-aging, reduction or prevention of skin wrinkles, improvement of skin elasticity, or improvement of skin roughness.

14. The use of claim 13, wherein the administration is applying to lips, scalp, or hair.

15. The multiple ceramides composition according to any one of claims 1-10 for use in treating, improving, or preventing a skin disease caused by decrease in skin barrier function.
